# EUROPÄISCHE PATENTSCHRIFT

(11) **EP 1 051 212 B1**
(45) Veröffentlichungstag und Bekanntmachung des Hinweises auf die Patenterteilung: **17.09.2003**
(21) Anmeldenummer: 98966814.0
(22) Anmeldetag: 22.12.1998
(51) Int. Cl.: A61M 15/00

(54) **INHALATIONSGERÄT FÜR PULVERFÖRMIGE MEDIKAMENTE**
INHALATION APPARATUS FOR POWDER MEDICATIONS
APPAREIL D'INHALATION DESTINE A DES MEDICAMENTS PULVERULENTS

(30) Priorität: 30.01.1998 DE 19804888; 29.05.1998 DE 19825434
(43) Veröffentlichungstag der Anmeldung: 15.11.2000
(73) Patentinhaber: Hagepharm GmbH, 55126 Mainz-Finthen (DE)
(72) Erfinder: GOLDEMANN, Raul, D-12203 Berlin (DE); SCHWARZWALD, Detlef, D-12051 Berlin (DE)
(74) Vertreter: Wablat, Wolfgang, Dr.Dr.
(86) Internationale Anmeldenummer: DE9803808
(87) Internationale Veröffentlichungsnummer: WO99038555

(56) Entgegenhaltungen:
- WO-A-92/05823
- WO-A-92/08509

## Beschreibung

Die Erfindung betrifft ein Inhalationsgerät für pulverförmige Medikamente, bestehend aus einem Gehäuse mit einem der Öffnung eines in dem Gehäuse befindlichen Pulverreservoirs zugeordneten, manuell betätigbaren und eine periphere Dosierkavität zur Aufnahme einer Pulverdosis aufweisenden drehbaren Dosiervorrichtung mit kreisbogenförmiger Umfangsfläche sowie einem in Höhe der Dosiervorrichtung an das Gehäuse angeschlossenem Mundstück und diesem gegenüberliegenden Lufteintrittsöffnungen zur Ausbildung eines Luftkanals, in dem die durch Drehen der Dosiervorrichtung freigesetzte Pulverdosis in den Atemluftstrom eines Benutzers abgegeben wird.

Ein derartiges, ohne Treibgas bzw. Zusatzluft betriebenes Inhalationsgerät wird beispielsweise in der EP 0 559 663 beschrieben. Bei diesem Pulverinhalator ist innerhalb eines Gehäuses eine mit einem pulverförmigen Medikament gefüllte Vorratskammer angeordnet, deren trichterförmige Öffnung durch eine von Hand drehbare Dosiertrommel verschlossen ist. Am Umfang der Dosiertrommel ist mindestens eine Dosierkavität ausgebildet, deren Größe der zu inhalierenden Pulverdosis entspricht und die mit dem Medikament gefüllt wird, wenn sie sich im Bereich der Öffnung des Pulverreservoirs befindet. In Höhe der Dosiervorrichtung ist an das Gehäuse ein Mundstück angeschlossen, dem auf der gegenüberliegenden Gehäuseseite Lufteintrittsöffnungen gegenüberstehen. Beim Drehen der Dosiertrommel fällt das pulverförmige Medikament aufgrund der Schwerkraft und gegebenenfalls unterstützt durch einen Rüttelmechanismus in den Luftkanal des Mundstücks und wird über den Einatmungsluftstrom vom Patienten inhaliert. Das bei derartigen Geräten bekanntermaßen kritische vollständige Entleeren der Dosierkavität und des Mundstücks, d. h. die zuverlässige Dispergierung oder Dosierung des pulverförmigen Medikaments, soll hier durch eine besondere Luftführung erreicht werden, die ein Ausblasen der Dosierkammer und durch einen kurzen Luftweg ein vollständiges Inhalieren der Pulverdosis bewirkt.

Die Inhalationsgeräte der beschriebenen Art sind jedoch insofern nachteilig, als das Medikament bei nur geringem Einatmungsluftstrom nicht vollständig ausgeblasen wird oder im Luftstrom nicht ausreichend verteilt wird, so daß eine zuverlässige Dosierung nicht gewährleistet ist. Die Dosierzuverlässigkeit ist zudem auch dadurch eingeschränkt, daß bei unvollständiger oder auch nicht erfolgter Einatmung das Medikament zwar bei der manuellen Betätigung der Dosiervorrichtung in das Mundstück gelangt, aber ganz oder teilweise in diesem verbleibt und bei einem weiteren Inhalationsvorgang demzufolge eine Überoder Mehrfachdosierung erfolgt.

Zur Verstärkung des Einatmungsluftstroms und einer damit verbundenen besseren Verteilung und zuverlässigeren Einnahme des Medikaments wurden andererseits bereits Pulverinhalatoren vorgeschlagen, die über eine Pumpeneinrichtung zur Erzeugung eines zusätzlichen Druckluftstroms verfügen.

Beispielsweise wird bei einem in der EP 0 549 605B1 beschriebenen treibgasfrei arbeitenden Inhalationsgerät mittels eines in einem Pulverreservoir durch Betätigung einer Taste bewegbaren Stempels mit einer eine Dosierkammer bildenden seitlichen Ausnehmung eine vorgegebene Dosis einer pulverförmigen medizinischen Substanz in den Strömungskanal des seitlich am Gerät vorgesehenen Mundstücks gebracht. Gleichzeitig wird dabei eine Innenwand der Dosierkavität an den Zylinderraum einer Pumpe angeschlossen. Mittels einer durch das Einatmen betätigten Schalteinrichtung wird der vorgespannte Kolben der Pumpe freigesetzt, um dabei einen Luftstrom zu erzeugen, der die pulverförmige medizinische Substanz über eine Düse in den Einatmungs-Luftstrom einträgt und in dieser verteilt.

Dieses Inhalationsgerät besteht aufgrund kompliziert ausgebildeter Mechanismen zur Dosierung des Medikaments und zur Erzeugung und Auslösung des zusätzlichen Druckluftstroms aus einer Vielzahl unterschiedlichster Einzelteile. Derartige Inhalatoren sind daher mit Bezug auf die Fertigung der Einzelteile und die Montage sehr kostenaufwendig und im Hinblick auf den komplizierten Aufbau und das notwendige Zusammenwirken unterschiedlicher Einzelelemente, insbesondere beim Eindringen von Staub, Schmutz und Atemfeuchtigkeit, auch störanfällig. Zudem ist die vollständige Befüllung der eine seitliche Ausnehmung bildenden Dosierkavität nicht mit Sicherheit oder nur mit zusätzlichen Bauteilen gewährleistet. Durch die Erzeugung des Zusatzluftstroms aufgrund der Kolbenbewegung der Pumpe und dessen Zuführung über einen Siebboden in die Dosierkavität ist der auf das pulverförmige Medikament ausgeübte notwendige Blasdruck schwach bzw. erfordert einen kräftig ausgelegten Pumpenantrieb.

Nachteilig ist jedoch auch bei dieser Lösung in entscheidendem Maße, daß sich das pulverförmige Medikament bereits vor der atemsynchronen Auslösung des Druckluftstroms in dem Mundstück befindet und bei nicht erfolgtem Einatmen auch dort verbleibt. Dadurch ist zum einen bei späterer Medikamenteneinnahme eine Doppeldosierung möglich, und andererseits kann das freiliegende Medikament verschmutzen oder feucht werden und dadurch die Funktionsfähigkeit des Pulverinhalators beeinträchtigen. Außerdem sind die bekannten Pulverinhalatoren in nicht ausreichendem Maß gegen das Eindringen von Schmutz und Feuchtigkeit, insbesondere Atemfeuchtigkeit, oder versehentliches Freisetzen des pulverförmigen Medikaments geschützt.

Der Erfindung liegt daher die Aufgabe zugrunde, ein Inhalationsgerät der eingangs erwähnten Art bereitzustellen, daß einfach aufgebaut und handhabungssicher ist und kostengünstig gefertigt werden kann und darüber hinaus eine exakte Dosierung und vollständige Inhalation des pulverförmigen Medikaments gewährleistet sowie eine Über- oder Mehrfachdosierung mit Sicherheit ausschließt.

Erfindungsgemäß wird die Aufgabe bei einem Inhalationsgerät gemäß dem Oberbegriff des Patentanspruches 1 in der Weise gelöst, daß die Dosiervorrichtung mit elastischen Spannmitteln verbunden ist sowie ein Anschlagstück aufweist und bei im Bereich der Pulverentnahmeöffnung des Pulverreservoirs verbleibender Dosierkavität bis an einen beweglichen Anschlag vorspannbar ist, wobei der Anschlag zur Freigabe der vorgespannten Dosiervorrichtung durch den Atemluftstrom des Benutzers bewegbar ist und die dadurch ausgelöste Beschleunigung der Dosiervorrichtung durch das Anschlagstück plötzlich unterbrechbar ist.

Der Grundgedanke der Erfindung besteht mit anderen Worten darin, daß die Dosiervorrichtung vor der Einnahme des Medikaments bzw. bevor die Dosierkavität mit dem Medikament in den Luftkanal gelangt, vorgespannt wird und in diesem vorgespannten Zustand an einem durch das Einatmen bewegbaren Anschlag gehalten wird. Mit dem Einatmen wird die Dosiervorrichtung freigesetzt und beschleunigt, wobei die beschleunigte Bewegung abrupt unterbrochen wird, indem das Anschlagstück der Dosiervorrichtung an das Gehäuse bzw. an den Gehäuseboden anschlägt. Diese plötzliche Unterbrechung der Drehbewegung der Dosiervorrichtung hat zur Folge, daß das pulverförmige Medikament mit hoher Geschwindigkeit aus der Dosierkavität herausgeschleudert wird und weiträumig im Luftkanal verteilt wird. Zum gleichen Zeitpunkt ist auch der Einatmungsluftstrom des Benutzers, der die Freisetzung der Dosierkavität und des Medikaments bewirkt hat, wirksam, so daß die feinverteilte Pulverdosis unmittelbar in den Atemluftstrom aufgenommen und im wesentlichen vollständig über die Atemwege in den Körper des Benutzers eingetragen werden kann. Da die Freisetzung des Medikaments durch die Atmung ausgelöst wird, muß das Medikament zwangsläufig auch inhaliert werden. Eine Über- oder Mehrfachdosierung bei einer nachfolgenden Betätigung des Gerätes durch im Mundstück verbliebendes Restpulver aus einem vorangegangenen Inhalationsversuch ist somit ausgeschlossen.

Nach einem weiteren Merkmal der Erfindung ist die Dosiervorrichtung als Dosierkugel ausgebildet. Dadurch wird die Luftströmung im Luftkanal in vorteilhafter Weise verwirbelt und die Pulververteilung weiter verbessert.

Gemäß einem anderen wesentlichen Erfindungsmerkmal ist der die Dosierkugel in vorgespanntem Zustand haltende bewegliche Anschlag an einer im Luftkanal schwenkbar aufgehängten, den Luftkanal verschließenden Auslöseklappe angebracht. Durch das Einatmen des Benutzers wird ein Unterdruck erzeugt, der die Auslöseklappe nebst Anschlag verschwenkt und so die Drehbewegung der vorgespannten Dosierkugel freigibt. Die schwenkbare Auslöseklappe schützt zudem das Gehäuseinnere beim versehentlichen Ausatmen in das Mundstück vor dem Eindringen von Feuchtigkeit.

In vorteilhafter Weiterbildung der Erfindung ist an der Außenseite des Gehäuses ein Betätigungshebel zum Drehen und Vorspannen der Dosierkugel angebracht, auf dem eine Abdeckkappe zum Verschließen des Mundstückes des Inhalationsgerätes auf Schienen verschiebbar befestigt ist. Die Abdeckkappe bleibt somit immer mit dem Gerät verbunden und kann nicht verloren gehen. Folglich ist die Verschmutzungsgefahr oder ein versehentliches Bewegen des derart arretierten Betätigungshebels gering bzw. ausgeschlossen.

Gemäß einem weiteren wichtigen Merkmal zur Ausgestaltung der Erfindung ist in dem Gehäuse eine Pumpeneinrichtung untergebracht, deren Druckluftreservoir über eine Ventileinrichtung an die Dosierkavität angeschlossen ist, und zwar derart, daß mit der atmungssynchronen Freigabe der vorgespannten Dosierkugel, d. h. der Freisetzung der Pulverdosis aus dem Pulverreservoir, gleichzeitig die Ventileinrichtung die Druckluftzufuhr auf die Dosierkavität bzw. das in dieser befindliche Medikament freigibt. Mit dieser Maßnahme wird die Dispersion des pulverförmigen Medikaments insbesondere in der frühen Phase der Deagglomeratron weiter verbessert. Außerdem ist dadurch eine zuverlässige und vollständige Einnahme des Medikaments vor allem bei Benutzern mit verminderter Atmungskapazität, wie älteren oder kranken Menschen oder Kindern, gewährleistet.

Weitere Merkmale, zweckmäßige Ausgestaltungen und Vorteile der Erfindung ergeben sich aus der nachfolgenden Beschreibung einer beispielhaften Ausführungsform der Erfindung.

Zwei Ausführungsbeispiele der Erfindung werden anhand der beigefügten Zeichnung näher erläutert. Es zeigen:
- Fig. 1: ein erfindungsgemäßes Inhalationsgerät in einer seitlichen Schnittansicht in der geschlossenen Ausgangslage, das mit Mitteln zur Erzeugung eines zusätzlichen Druckluftstroms ausgerüstet ist;
- Fig. 2: das Inhalationsgerät nach Fig. 1, jedoch mit von dessen Mundstück abgezogener und nach unten verschwenkter Abdeckkappe;
- Fig. 3: das Inhalationsgerät nach Fig. 1 und 2 in einer schematischen Schnittdarstellung zum Zeitpunkt des Inhalierens der pulverförmigen medizinischen Substanz;
- Fig. 4: eine seitliche Schnittansicht eines erfindungsgemäßen Inhalationsgerätes in einer geschlossenen Ausgangslage, das ohne einen zusätzlichen Druckluftstrom arbeitet;
- Fig. 5: ein Inhalationsgerät mit einem in das Mundstück eingebundenen Nasenadapter und
- Fig. 6: eine Seitenansicht des Pulverinhalators in drei unterschiedlichen Stellungen der Abdeckkappe.

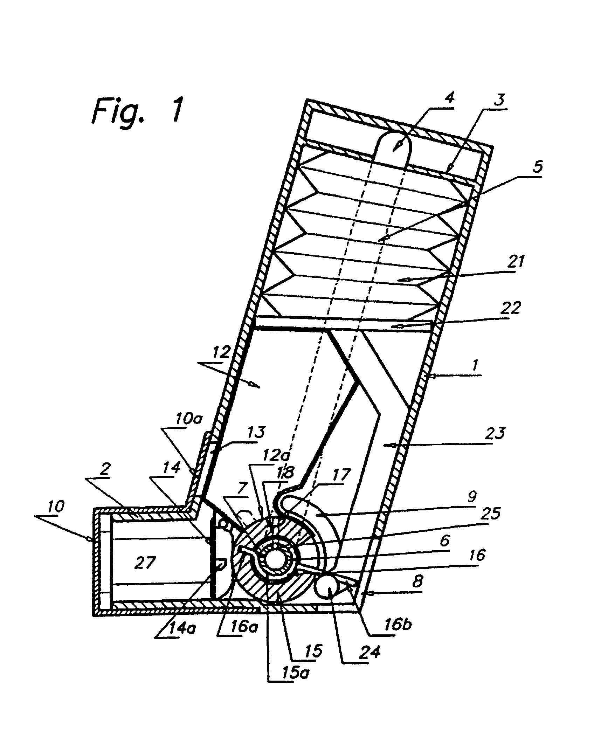

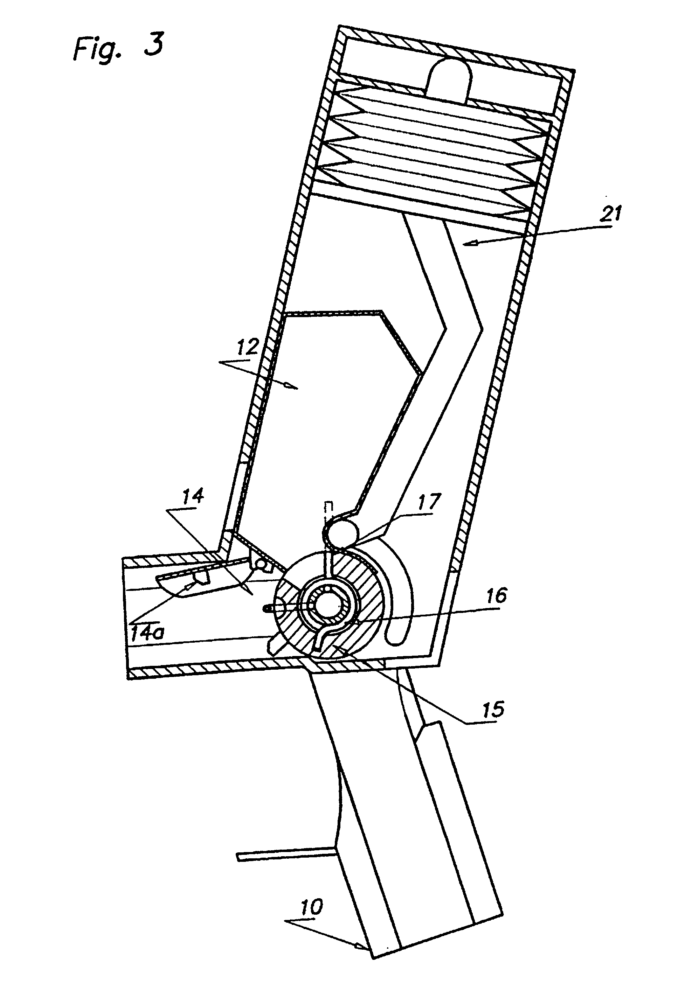

Das Pulver-Inhalationsgerät mit Druckluftzufuhr gemäß den Fig. 1 bis 3 umfaßt ein längliches Gehäuse 1 mit einem an dessen unterem Ende seitlich angeformten Mundstück 2 und in dessen oberem Bereich ausgebildeter Querwand 3 mit einer Luftauslaßöffnung 4, die an einen an der Innenwand des Gehäuses 1 in dessen unteren Bereich führenden Druckluftkanal 5 angeschlossen ist. In seinem unteren Teil weist das Gehäuse 1 einen in das Gehäuseinnere gerichteten, hohl ausgebildeten Achszapfen 6 sowie eine axial mit dem Achszapfen 6 fluchtende, jedoch von der Außenwand des Gehäuses 1 abstrebende Achsnabe (in der Zeichnung nicht sichtbar) auf. Die Achsnabe und der Achszapfen sind etwa in Höhe der Längsachse des Mundstücks 2, aber quer zu dieser, an die Gehäusewand angeformt. In der Wandung des hohlen Achszapfens 6 befindet sich auf der Seite des Mundstücks 2 eine erste radiale Achszapfenbohrung 7. Der in dem Achszapfen 6 gebildete Hohlraum ist an den Druckluftkanal 5 angeschlossen. Außerdem befinden sich in der dem Mundstück 2 gegenüberliegenden Rückwand des Gehäuses 1 Lufteintrittsöffnungen 8, und in der Seitenwand des Gehäuses 1 ist ein bogenförmiges Langloch 9 ausgebildet.

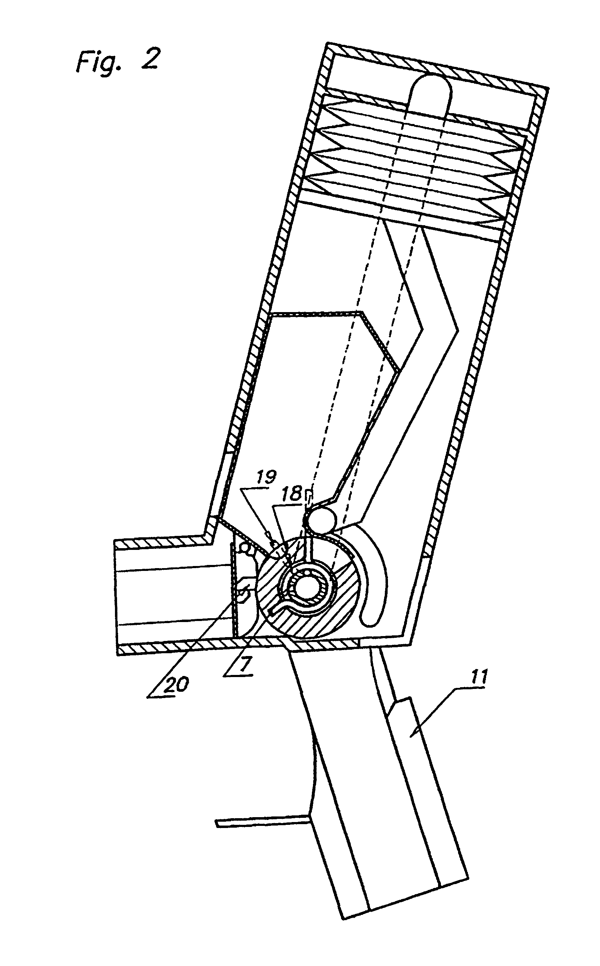

Auf dem Mundstück 2 ist in Führungen (nicht dargestellt) eine Abdeckkappe 10 verschiebbar angeordnet, die teleskopisch mit einem an seinem freien Ende an der oben erwähnten Achsnabe (nicht dargestellt) drehbar gelagerten Betätigungshebel 11 (Fig. 2) verbunden ist. Der Betätigungshebel 11 verfügt außerdem über eine von einem Drehteil an seiner Schwenkachse ausgehende Verlängerung (nicht dargestellt), deren freies Ende über das bogenförmige Langloch 9 und einen Mitnehmerbolzen 24 mit einem im Innern des Gehäuses 1 angeordneten Winkelhebel 23 in Wirkverbindung steht. Durch die teleskopische Anbindung der Abdeckkappe 10 an den am Gehäuse 1 angelenkten Betätigungshebel 11 kann diese zwar vom Mundstück 2 abgezogen werden, bleibt aber dennoch mit dem Inhalationsgerät verbunden und kann somit nicht verlorengehen.

In dem Gehäuse 1 ist oberhalb des Achszapfens 6 ein Pulverreservoir 12 angebracht, das über eine zum Achszapfen 6 weisende Pulverentnahmeöffnung 12a verfügt. In der zum Pulverreservoir 12 gerichteten Wand des Gehäuses 1 befindet sich ein Sichtfenster 13 zur Füllstandskontrolle. Das Pulverreservoir 12 besteht aus einem transparenten Werkstoff. Zur Vermeidung von Lichteinflüssen auf das Medikament ist an die Abdeckkappe 10 eine Scheibe 10a angeformt, um das Sichtfenster 13 bei aufgeschobener Abdeckkappe 10 abzudecken.

Der Innenraum des Gehäuses 1 ist zur Öffnung des Mundstücks 2 hin durch eine gelenkig befestigte Auslöseklappe 14 verschlossen, die nach oben und außen verschwenkbar ist. An der zum Gehäuseinneren gerichteten Seite der Auslöseklappe 14 ist ein Anschlag 14a angebracht.

Die Pulverentnahmeöffnung 12a ist durch eine an deren Rand bzw. verlängerten Randbereichen anliegende Dosierkugel 15 dicht verschlossen. Die Dosierkugel 15 weist eine mittige Lagerbohrung 15a auf, in der sie auf dem Achszapfen 6 drehbar gelagert ist. Auf dem Achszapfen 6 ist außerdem eine Torsionsfeder 16 mit einem kurzen feststehenden Schenkel 16a und einem langen bewegbaren Schenkel 16b gelagert, wobei der kurze Schenkel 16a an der Dosierkugel 15 gehalten ist.

Die Dosierkugel 15 verfügt an ihrer dem Pulverreservoir 12 zugewandten Umfangsfläche über eine halbkugelförmige Dosierkavität 17 zur Aufnahme des pulverförmigen Medikaments, und zwar, wenn diese sich im Bereich der Pulverentnahmeöffnung 12a befindet.

In der Dosierkugel 15 ist außerdem eine mit der ersten radialen Achszapfenbohrung 7 in derselben senkrechten Schnittebene liegende radiale Ventilbohrung 18 ausgebildet. An die Öffnung der Ventilbohrung 18 ist am Umfang der Dosierkugel 15 ein Blasrohr 19 angeschlossen, dessen offene Seite auf die Dosierkavität 17 gerichtet ist. Schließlich ist an der Umfangsfläche der Dosierkugel 15 ein Anschlagstück 20 ausgebildet, um die Dosierkugel 15 entweder am Anschlag 14a der Auslöseklappe 14 oder am Boden des Gehäuses 1 zu arretieren.

Unterhalb der Querwand 3 ist ein Faltenbalg 21 angeordnet, der eine mit der Luftauslaßöffnung 4 in Verbindung stehende Öffnung aufweist. Auf der gegenüberliegenden Seite ruht der Faltenbalg 21 auf einer Stützplatte 22, die an der Unterseite mit dem oben erwähnten, als Druckstab wirkenden Winkelhebel 23 verbunden ist. Das freie Ende des Winkelhebels 23, das über das Langloch 9 außerhalb des Gehäuses 1 mit der Verlängerung (nicht dargestellt) des Betätigungshebels 11 verbunden ist, weist einen rechtwinklig von diesem abstrebenden Mitnehmerbolzen 24 auf, der mit dem langen Schenkel 16 b der Torsionsfeder 16 in Wirkverbindung steht.

Die Funktion der oben in statischem Zustand erläuterten Ausführungsform eines Pulverinhalators mit Druckluftzufuhr wird nachfolgend beschrieben:

In unbenutztem Zustand des Pulverinhalators befindet sich die Abdeckkappe 10 in der auf das Mundstück 2 geschobenen Lage (Fig. 1), so daß in das Gerät keine Fremdkörper eindringen können. Ein versehentliches Öffnen oder ein Verlieren der Abdeckkappe 10 oder gar ein unbeabsichtigtes Auslösen der Abgabe des Medikaments, beispielsweise bei der Unterbringung in einer Tragetasche, Jackentasche und dergleichen, ist somit nicht möglich.

Das Sichtfenster 13 ist durch die Scheibe 10 a abgedeckt und schützt das pulverförmige Medikament in dem transparenten Pulverreservoir 12 vor Lichteinwirkung. Die Auslöseklappe 14 befindet sich in der senkrechten, das Mundstück 2 verschließenden Lage. Der Faltenbalg 21 und die Torsionsfeder 16 sind entsprechend der unteren Stellung des Winkelhebels 23 in entspanntem Zustand, und die Dosierkavität 17 der Dosierkugel 15 liegt in der pulverförmigen Substanz, und zwar auf der in der Zeichnung rechten Seite der trichterförmigen Pulverentnahmeöffnung 12a. Das Pulverreservoir 12 ist für eine Einmalfüllung von etwa 200 Dosierungen ausgebildet.

Zur Entnahme einer durch die Größe der Dosierkavität 17 bestimmten Dosis des pulverförmigen Medikaments wird die Abdeckkappe 10 vom Mundstück 12 abgezogen, verbleibt aber mit dem Betätigungshebel 11 und damit mit dem Gerät in unlösbarer Verbindung. Beim Herunterschwenken des Betätigungshebels 11 wird der Mitnehmerbolzen 24 entlang dem bogenförmigen Langloch 9 in die obere Lage verschwenkt und damit der mit dem Betätigungshebel 11 verbundene Winkelhebel 23 nach oben gedrückt, um einerseits den Faltenbalg 21 zusammenzupressen und damit die in diesem befindliche Luft zu verdichten und andererseits die Dosierkugel 15 mit Hilfe der Torsionsfeder 16 zunächst bis zur Berührung ihres Anschlagstückes 20 mit dem Anschlag 14a der Auslöseklappe 14 zu drehen und - vom Zeitpunkt des Anschlagens an - die Torsionsfeder 16 zu spannen und damit die Dosierkugel 15 vorzuspannen. Die Dosierkavität 17 befindet sich in vorgespanntem Zustand der Dosierkugel 15 am in der Zeichnung linken Rand der Pulverentnahmeöffnung 12a.

Beim Einatmen des Benutzers über das Mundstück 2 wird nun aufgrund des entstehenden Unterdrucks die Auslöseklappe 14 nach oben verschwenkt und damit die am Anschlag 14a gehaltene Dosierkugel 15 freigegeben, die sich unter der Wirkung der Federkraft der Torsionsfeder 16 bis zum hörbaren Anstoßen des Anschlagstückes 20 am Boden des Gehäuses 1 weiterdreht. Gleichzeitig kommt die radiale Ventilbohrung 18 in der Dosierkugel 15 in eine Lage, in der sie in einer Flucht mit der ersten radialen Achszapfenbohrung 7 des hohlen Achszapfens 6 liegt, so daß die im Faltenbalg 21 verdichtete Luft über die Luftauslaßöffnung 4, den Druckluftkanal 5, den Hohlraum des Achszapfens 6, die radiale Achszapfenbohrung 7, die Ventilbohrung 18 und das Blasrohr 19 stoßartig in die sich jetzt außerhalb des Pulverreservoir 12 befindliche und mit dem pulverförmigen Medikament exakt gefüllte Dosierkavität 17 geblasen wird.

Das pulverförmige Medikament wird durch den eingeblasenen Druckluftstrom und durch die ruckartige Unterbrechung der Drehbewegung der Dosierkugel 15 sowie die Flieh- und Schwerkräfte in dem Einatmungs-Luftstrom des Benutzers verwirbelt und gelangt - im Luftstrom fein verteilt - in die Atemwege des Benutzers. Gleichzeitig wird die Pulververnebelung durch den Druckluftstrom verstärkt, so daß auch bei einem unterhalb des Durchschnittswertes von 1 l/s liegenden Einatemvolumenstroms, z. B. bei Kindern oder älteren Menschen, die sichere Zuführung des pulverförmigen Medikaments in die Atemwege gewährleistet ist. Unmittelbar nach dem Einatmen fällt die Auslöseklappe 14 wieder in die senkrechte Ausgangslage, so daß beim versehentlichen Ausatmen in das Mundstück 12 keine Atemluftfeuchtigkeit an die Dosierkugel 15 bzw. an die pulverförmige Substanz gelangen kann. Die Zurückstellung der Auslöseklappe in die senkrechte Ausgangslage wird zusätzlich durch einen in der Abdeckkappe befindlichen, beim Schließen der Abdeckkappe auf die Auslöseklappe wirkenden Steg sichergestellt (nicht gezeigt).

Durch Verschwenken des Betätigungshebels 11 in die Schließstellung der Abdeckkappe 10 werden der Winkelhebel 23 nebst Faltenbalg 21 und die Dosierkugel 15 sowie die Torsionsfeder 16 wieder in ihre in Fig. 1 dargestellte Ausgangslage gebracht, wobei das aus der Achszapfenbohrung 7 und der Ventilbohrung 18 bzw. der Mantelfläche der zentralen Lagerbohrung 15a der Dosierkugel 15 gebildete Druckluft-Zuführungsventil wieder geschlossen ist. In dem hohlen Achszapfen 6 befindet sich eine zweite radiale Achszapfenbohrung 25 für den Lufteinlaß in den Faltenbalg 21. Wenn sich die Dosierkugel 15 in der Ausgangslage befindet, liegt die zweite radiale Achszapfenbohrung 25 mit der Ventilbohrung 18 (oder einer weiteren, nicht dargestellten Bohrung in einer Flucht, so daß in den Faltenbalg 21 Luft für einen weiteren Kompressionsvorgang nachströmen kann.

Erst nach einem erneuten Öffnen der Abdeckkappe 10 und Verschwenken des Betätigungshebels 11 kann der Pulverinhalator aktiviert werden und danach, jedoch nur über den Einatmungs-Luftstrom des Benutzers - eine weitere Medikamentdosis in die Atemwege gelangen. Sofern der Benutzer nicht einatmet und/oder das Mundstück 2 wieder verschließt, verbleibt das pulverförmige Medikament in der Dosierkavität 17 innerhalb des Pulverreservoirs 12. Eine versehentliche Doppel- oder Mehrfachdosierung ist daher ausgeschlossen. Zudem wird durch das Anschlagen des Anschlagstückes 20 der Dosierkugel 15 am Gehäuseboden und die aus dem Blasrohr 19 unter Zischen ausströmende Fremdluft ein Geräusch erzeugt, das dem Benutzer die tatsächliche Einnahme des pulverförmigen Medikament signalisiert.

Das Inhalationsgerät ist somit auch von weniger geschickten Benutzern auf einfache Weise und sicher handhabbar. Da seine wenigen Einzelteile überwiegend aus Spritzgußteilen gefertigt werden, kann es durch Montage in einem zweiteilig ausgebildeten Gehäuse auf einfache Weise und kostengünstig hergestellt werden.

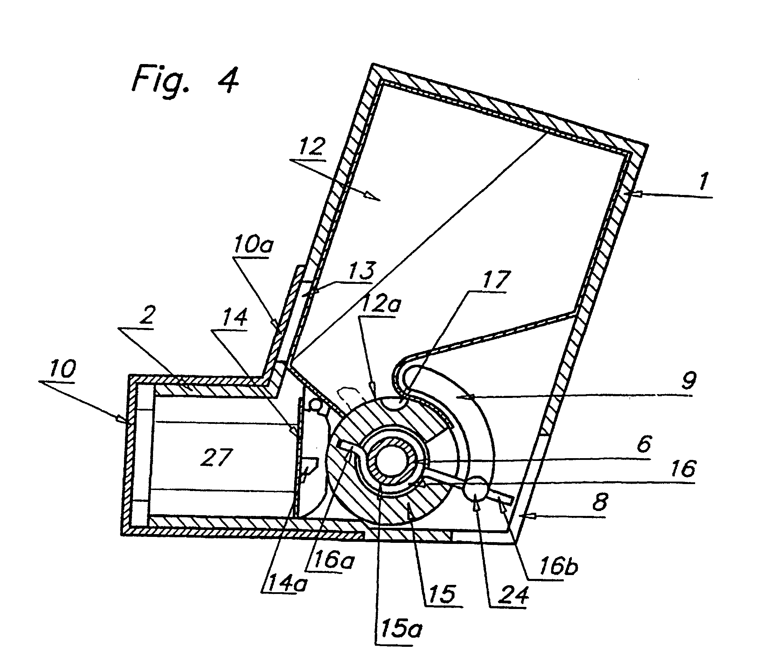

Fig. 4 zeigt das erfindungsgemäße Inhalationsgerät in einer vereinfachten Ausführungsform, und zwar ohne Verwendung der zuvor beschriebenen Mittel zur Erzeugung eines zusätzlichen Druckluftstrom. Diese Ausführungsvariante ist durch einen unkomplizierten Aufbau und durch eine einfache Herstellung gekennzeichnet und gewährleistet auch ohne Zusatzluft eine gute Verteilung des Pulvermedikaments im Atemluftstrom und eine vollständige und zuverlässige Einnahme durch den Patienten. In der in Fig. 1 dargestellten Ausgangslage mit durch die Abdeckkappe 10 verschlossenem Mundstück 2 befindet sich die Dosierkugel 15 in der entspannten Lage der mit ihr verbundenen Torsionsfeder 16. Der mit dem langen Schenkel 16b der Torsionsfeder 16 in Wirkverbindung stehende Mitnehmerbolzen 24 des Betätigungshebels 11 ist im Langloch 9 in der unteren Position. Wenn die Abdeckkappe 10 vom Mundstück 2 abgezogen und anschließend der Betätigungshebel 11 in die in Fig. 2 gezeigten Stellung verschwenkt wird, dreht sich mit der Bewegung des Mitnehmerbolzens 24 gleichzeitig die Dosierkugel 15, und zwar solange, bis das Anschlagstück 20 der Dosierkugel 15 am Anschlag 14a zur Anlage kommt. Bei der Weiterbewegung des Betätigungshebels 11 bis zum Anschlagen seines Mitnehmerbolzens 24 am anderen, oberen Ende des Langloches 9 wird die Torsionsfeder 16 gespannt und damit die Dosierkugel 15, deren Dosierkavität 17 jetzt unmittelbar am Rand der Öffnung des Pulverreservoirs 12 steht, vorgespannt. Beim Einatmen und dem durch den entsprechenden Unterdruck im Mundstück bedingten Hochschwenken der Auslöseklappe 14 wird die Dosierkugel 15 plötzlich freigegeben. Die daraufhin aufgrund der Vorspannung bedingte Beschleunigung der Dosierkugel wird anschließend durch Anschlagen des Anschlagstückes 20 am Boden des Mundstückes 2 ruckartig unterbrochen.

Die plötzliche Beschleunigung und Unterbrechung der Bewegung des in der Dosierkavität 17 befindlichen pulverförmigen Medikaments und die Fliehkräfte bewirken das vollständige Lösen des Medikaments aus der Dosierkavität 17 und dessen breite Verteilung und Verwirbelung in einem großen Bereich des Luftkanals im Mundstück 2. Durch die gleichzeitige - aufgrund des Unterdrucks impulsartige - Zuführung der Atemluft ist es somit möglich, auch ohne einen zusätzlich erzeugten Druckluftstrom mit einer Pumpeneinrichtung eine vollständige Zuführung der jeweils entnommenen Pulverdosis zu den betreffenden Organen des Benutzers zu gewährleisten.

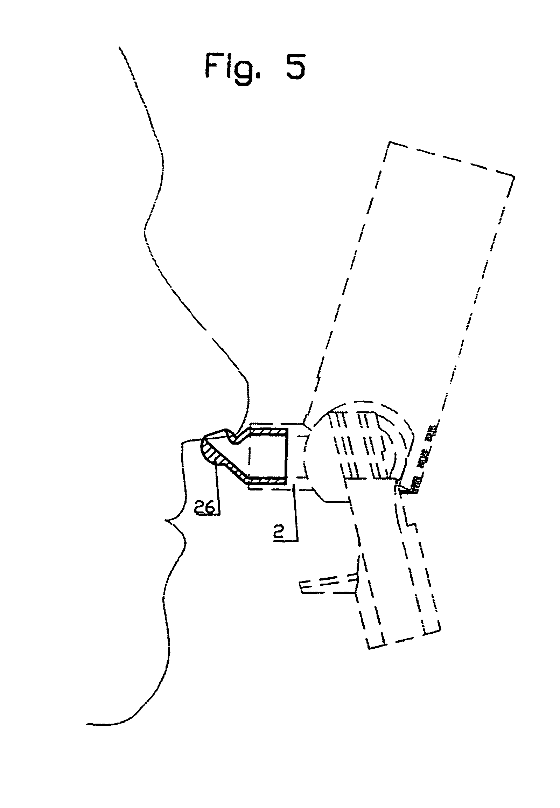

In Fig. 5 ist eine Ausführungsvariante des Pulverinhalators mit einem in das Mundstück 2 eingesetzten monorhinalen Nasenadapter 26 wiedergegeben. Der Nasenadapter 26 kann fest oder lösbar angebracht sein und auch nachträglich, und zwar unlösbar, in das Mundstück eingesteckt werden. Der Nasenadapter 26 ist zudem so ausgebildet, daß er nur zu einem bestimmten Inhalationsgerät paßt.

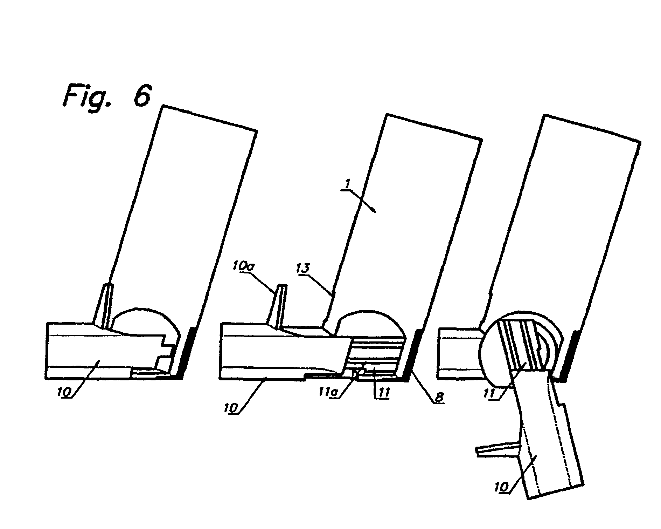

Fig. 6 zeigt das Inhalationsgerät mit vollständig auf das Mundstück 2 aufgeschobener Abdeckkappe 10, mit vom Mundstück 2 abgezogener Abdeckkappe 10 und schließlich mit nach unten verschwenktem, das Inhalationsgerät aktivierendem Betätigungshebel 11. Aus Fig. 5 wird deutlich, daß die Abdeckkappe 10 auf einem Schienenprofil 11a auf dem Betätigungshebel 11 geführt ist und den Betätigungshebel 11 teleskopisch verlängert, aber mit diesem immer verbunden bleibt.

Mit Hilfe des erfindungsgemäßen Inhalationsgerätes lassen sich alle pulverförmigen Medikamente verabfolgen. Als besonders vorteilhaft hat es sich in der Behandlung von asthmatischen Erkrankungen und dergleichen erwiesen.

Als zu verabreichende Wirkstoffe kommen beispielsweise Betasympatomimetika und Corticoide in Betracht. Insbesondere werden die Substanzen Salbutamol, Dinatriumcromoglykat, Budesonid, Beclometason, Reproterol, Fenoterol sowie auch Kombinationen/Mischungen dieser Substanzen genannt.

### Bezugszeichenliste

- 1: Gehäuse
- 2: Mundstück
- 3: Querwand
- 4: Luftaustrittsöffnung
- 5: Druckluftkanal
- 6: hohler Achszapfen
- 7: erste radiale Achszapfenbohrung (für Luftauslaß)
- 8: Lufteintrittsöffnungen
- 9: bogenförmiges Langloch
- 10: Abdeckkappe
- 10a: Scheibe
- 11: Betätigungshebel
- 11a: Schienenprofil
- 12: Pulverreservoir
- 12a: Pulverentnahmeöffnung
- 13: Sichtfenster
- 14: Auslöseklappe
- 14a: Anschlag
- 15: Dosierkugel
- 15a: Lagerbohrung
- 16: Torsionsfeder
- 16a: kurzer Schenkel von 16
- 16b: langer Schenkel von 16
- 17: Dosierkavität
- 18: Ventilbohrung in 15
- 19: Blasrohr
- 20: Anschlagstück von 15
- 21: Faltenbalg
- 22: Stützplatte
- 23: Winkelhebel
- 24: Mitnehmerbolzen
- 25: zweite radiale Achszapfenbohrung (Lufteinlaß)
- 26: Nasenadapter
- 27: Luftleitkanal

## Patentansprüche

1. Inhalationgerät für pulverförmige Medikamente, bestehend aus einem Gehäuse (1) mit einem der Öffnung eines in dem Gehäuse befindlichen Pulverreservoirs (12) zugeordneten, manuell betätigbaren, und eine periphere Dosierkavität zur Aufnahme einer Pulverdosis aufweisenden, drehbaren Dosiervorrichtung (15) mit kreisbogenförmiger Umfangsfläche sowie einem in Höhe der Dosiervorrichtung (15) an das Gehäuse (1) angeschlossenem Mundstück (2) und diesem gegenüberliegenden Lufteintrittsöffnungen (4) zur Ausbildung eines Luftkanals (5), in dem die durch Drehen der Dosiervorrichtung (15) freigesetzte Pulverdosis in den Atemluftstrom eines Benutzers ab-gegeben wird, **dadurch gekennzeichnet, daß** die Dosiervorrichtung (15) mit elastischen Spannmitteln (16) verbunden ist sowie ein Anschlagstück (20) aufweist und bei im Bereich der Pulverentnahmeöffnung (12a) des Pulverreservoirs (12) verbleibender Dosierkavität (17) bis an einen beweglichen Anschlag (14a) vorspannbar ist, wobei der Anschlag (14a) zur Freigabe der vorgespannten Dosiervorrichtung (15) durch den Atemluftstrom des Benutzers bewegbar ist und die dadurch ausgelöste Beschleunigung der Dosiervorrichtung (15) durch das Anschlagstück (20) plötzlich unterbrechbar ist.

2. Inhalationgerät nach Anspruch 1, **dadurch gekennzeichnet, daß** die Dosiervorrichtung als Dosierkugel (15) mit einer zentrischen Lagerbohrung (15a) ausgebildet ist und um einen Achszapfen (6) drehbar gelagert ist.

3. Inhalationsgerät nach Anspruch 1 und 2, **dadurch gekennzeichnet, daß** das elastische Spannmittel eine auf dem Achszapfen drehbar gelagerte Torsionsfeder (16) mit einem kurzen (16a) und einem langen Schenkel (16b) ist, wobei der kurze Schenkel (16a) mit der Dosierkugel (15) und der lange Schenkel (16b) über ein bogenförmiges Langloch (9) im Gehäuse (1) mit einem an der Außenseite des Gehäuses (1) in axialer Flucht mit dem Achszapfen (6) drehbar gelagerten Betätigungshebel (11) zum Drehen und Vorspannen der Dosierkugel (15) verbunden ist.

4. Inhalationsgerät nach Anspruch 3, **dadurch gekennzeichnet**, der Betätigungshebel (11) über einen Mitnehmerbolzen (24) in dem Langloch (9) geführt und in vorgespannter Lage der Dosierkugel (15) durch Verrasten oder Überschreiten eines Totpunktes arretierbar ist.

5. Inhalationsgerät nach Anspruch 1, **dadurch gekennzeichnet, daß** der atemsynchron bewegliche Anschlag (14a) an einer den Luftleitkanal (27) verschließenden, im Gehäuse (1) auf der Mundstückseite unmittelbar vor der Dosierkugel (15) schwenkbar aufgehängten Auslöseklappe (14) angebracht ist und bei vorgespannter Dosierkugel (15) mit deren Anschlagstück (20) in Wirkverbindung steht, wobei die Dosierkugel (15) so im Luftleitkanal (27) angeordnet ist, **daß** deren Anschlagstück (20) nach Freigabe durch den beweglichen Anschlag (14a) unter der Wirkung der Federkraft der Torsionsfeder (16) am Boden des Gehäuses (1) gehalten ist und die Dosierkavität (17) außerhalb der Pulverentnahmeöffnung (12a) liegt.

6. Inhalationsgerät nach Anspruch 3 und 4, **dadurch gekennzeichnet, daß** an dem freien Ende des Betätigungshebels (11) eine Abdeckkappe (10) verschiebbar gehalten ist, die in der Ausgangsstellung des Betätigungshebels (11) auf das Mundstück (2) am Gehäuse (1) aufschiebbar ist.

7. Inhalationsgerät nach Anspruch 1, **dadurch gekennzeichnet, daß** das Pulverreservoir (12) aus transparentem Material besteht und im Gehäuse (1) im Bereich des unteren Teils des Pulverreservoirs (12) ein Sichtfenster (13) vorgesehen ist.

8. Inhalationsgerät nach Anspruch 6 und 7, **dadurch gekennzeichnet, daß** an der Abdeckkappe (10) eine Scheibe (10a) angebracht ist, die das Sichtfenster (13) bei aufgeschobener Abdeckkappe (10) verdeckt.

9. Inhalationsgerät nach einem der Ansprüche 1 bis 8, **dadurch gekennzeichnet, daß** die Dosierkavität (17) als kugelsegmentförmige Ausnehmung ausgebildet ist.

10. Inhalationsgerät nach einem der Ansprüche 1 bis 9, **dadurch gekennzeichnet, daß** die Dosierkugel austauschbar ist und die Dosierkugeln mit unterschiedlicher Größe der Dosierkavität (17) einsetzbar sind.

11. Inhalationsgerät nach einem der Ansprüche 1 bis 10, **dadurch gekennzeichnet, daß** an das Pulverreservoir (12) eine Rüttelvorrichtung angeschlossen ist.

12. Inhalationsgerät nach einem der Ansprüche 1 bis 11, **dadurch gekennzeichnet, daß** zur intranasalen Inhalation ein in das Mundstück (2) lösbar oder fest eingelegter Nasenadapter (26) vorgesehen ist.

13. Inhalationsgerät nach Anspruch 12, **dadurch gekennzeichnet, daß** der Nasenadapter eine aus der Öffnung des Mundstücks (2) herausragende Olive mit einem luftdicht an das Nasenloch anlegbaren Strömungskanal aufweist.

14. Inhalationsgerät nach Anspruch 12 und 13, **dadurch gekennzeichnet, daß** der Nasenadapter (26) im Mundstück (2) mit Rastmittel gehalten ist, die so ausgebildet sind, daß bestimmte Nasenadapter auf bestimmte Pulverinhalatoren abgestimmt sind.

15. Inhalationsgerät nach einem der Ansprüche 1 bis 14, **dadurch gekennzeichnet, daß** die Abdeckkappe (10) gegenüber der Ausführung ohne Nasenadapter (26) verlängert ist und über den Nasenadapter auf das Mundstück (2) aufschiebbar ist.

16. Inhalationsgerät nach einem der Ansprüche 1 bis 15, **dadurch gekennzeichnet, daß** das Pulverreservoir (12) für eine Einmalfüllung mit mindestens 200 Dosierungen ausgebildet ist.

17. Inhalationsgerät nach einem der Ansprüche 1 bis 16, **dadurch gekennzeichnet, daß** in dem Gerät vorzugsweise bei hygroskopischem pulverförmigem Medikament ein auf dieses wirkenden Trockenmittel vorgesehen ist.

18. Inhalationsgerät nach einem der Ansprüche 1 bis 17, **dadurch gekennzeichnet, daß** in dem Gehäuse (1) eine Pumpeneinrichtung (21, 22) untergebracht ist, deren Druckluftreservoir über eine Ventileinrichtung (7, 15a, 18, 25) oder ein Membranventil an die Dosierkavität (17) angeschlossen ist, derart, daß mit der atmungssynchronen Freigabe der vorgespannten Dosierkugel (15) vom beweglichen Anschlag (14a) gleichzeitig die Ventileinrichtung (7, 15a, 18, 25) die gespeicherte Druckluft freigibt.

19. Inhalationsgerät nach einem der Ansprüche 1 bis 18, **dadurch gekennzeichnet, daß** die Pumpeneinrichtung aus einem auf einer beweglichen Stützplatte (22) gehaltenen und auf der gegenüberliegenden Seite gegen eine Querwand (3) im Gehäuse (1) abgestützten Faltenbalg (21) gebildet ist und die Stützplatte (22) mit einem als Kraftübertragungsmittel dienenden Winkelhebel (23) verbunden ist, der an seinem gegenüberliegenden Ende an den Mitnehmerbolzen (24) des Betätigungshebels (11) angeschlossen ist, wobei der Faltenbalg (21) über eine Luftaustrittsöffnung (4) an einen Druckluftkanal (5) im Gehäuse (1) und eine radiale Achszapfenbohrung (25) mit einem im Achszapfen (6) ausgebildeten Hohlraum in Verbindung steht.

20. Inhalationsgerät nach einem der Ansprüche 1 bis 19, **dadurch gekennzeichnet, daß** die Ventileinrichtung zur atmungssynchron gesteuerten Druckluftzufuhr aus dem Faltenbalg (21) in den Luftleitkanal (27) aus der zweiten radialen Achszapfenbohrung (25) und einer ersten radialen Achszapfenbohrung (7), der Wandung der Lagerbohrung (15a) der Dosierkugel (15) und einer radialen Ventilbohrung (18) in der Dosierkugel (15) gebildet ist, wobei die Ventilbohrung (18) in der Anschlagstellung des Ventilstückes (20) am Boden des Gehäuses (1) mit der ersten radialen Achszapfenbohrung in einer Flucht liegt.

21. Inhalationsgerät nach einem der Ansprüche 1 bis 20, **dadurch gekennzeichnet, daß** an die Ventilbohrung (18) in der Umfangsfläche der Dosierkugel (15) ein gebogenes Blasrohr (19) angeschlossen ist, dessen Öffnung von außen in Richtung der Dosierkavität (17) weist.

22. Inhalationsgerät nach einem der Ansprüche 1 bis 21, **dadurch gekennzeichnet, daß** bis auf die Torsionsfeder (16) sämtliche Bauteile im Spritzgußverfahren aus Kunsstoff gefertigt sind.

## Claims

1. An inhalation apparatus for powder medications, consisting of a housing (1) with a circular rotatable dosing unit (15) that is associated with the opening of a powder reservoir (12) in the housing, can be operated manually, and comprises a peripheral dosing cavity (17) for receiving the powder dose, and with a mouthpiece (2) attached to the housing (1) level with the dosing unit (15) and air intake openings (4) on the opposite side of the mouthpiece to form an air duct (5) in which the powder dose that is released by turning the dosing unit (15) is discharged into a user's respiratory airflow, **characterized in that** the dosing unit (15) is connected to elastic chucking means (16) and comprises a stop piece (20), and that the dosing unit (15) can be preloaded to a movable stopper (14a) while the dosing cavity (17) remains in the area of the powder dispensing opening (12a) of the powder reservoir (12) and said stopper (14a) can be moved by the user's respiratory airflow to release the preloaded dosing unit (15) and that the acceleration of the dosing unit (15) triggered in this way can suddenly be interrupted by the stop piece (20).

2. The inhalation apparatus according to claim 1, **characterized in that** the dosing unit is shaped as a dosing ball (15) with a central bearing bore (15a) and is pivotably seated around an axle end (6).

3. The inhalation apparatus according to claims 1 and 2, **characterized** that the elastic chucking means is a torsion spring (16) seated pivotably on the axle end and comprising a short leg (16a) and a long leg (16b), the short leg (16a) being connected to the dosing unit (15) and the long leg (16b) being connected via a bow-shaped slot (9) in the housing (1) to an operating handle (11) for rotating and preloading the dosing ball (15) which is pivotably seated at the outside of the housing (1) in axial alignment with the axle end (6).

4. The inhalation apparatus according to claim 3, **characterized in that** the operating lever (11) is conducted over a tappet (24) in the slot (9) and can be locked when the dosing ball (15) is in preloaded position by interlocking or exceeding a dead center.

5. The inhalation apparatus according to claim 1, **characterized in that** the stopper (14a) that can be moved in sync with breathing is attached to a trip latch (14) hinged directly in front of the dosing ball (15) on the mouthpiece side in the housing (1) and closing the air duct (27), and **in that** said latch (14) is in a cooperative relationship with the stop piece (20) when the dosing ball (15) is preloaded as the dosing ball (15) is placed in the air duct (27) in such a way that its stop piece (20), when released by the movable stopper (14a), is held at the bottom of the housing (1) by the force of the torsion spring (16) and the dosing cavity (17) is located outside the powder dispensing opening (12a).

6. The inhalation apparatus according to claims 3 and 4, **characterized in that** a cap (10) is movably held on the free end of the operating lever (11) that can be slid onto the mouthpiece (2) on the housing (1) when the operating lever (11) is in its initial position.

7. The inhalation apparatus according to claim 1, **characterized in that** the powder reservoir (12) consists of a transparent material and that an inspection window (13) is provided in the housing (1) in the lower part (1) of the powder reservoir (12).

8. The inhalation apparatus according to claims 6 and 7, **characterized in that** a disk (10a) is attached to the cap (10) that covers the inspection window (13) when the cap (10) is slid on.

9. The inhalation apparatus according to any one of claims 1 through 8, **characterized in that** the dosing cavity (17) is a recess with the shape of a spherical segment.

10. The inhalation apparatus according to any one of claims 1 through 9, **characterized in that** the dosing ball is replaceable and that dosing balls with dosing cavities (17) of various sizes can be inserted.

11. The inhalation apparatus according to any one of claims 1 through 10, **characterized in that** a vibrator is connected to the powder reservoir (12).

12. The inhalation apparatus according to any one of claims 1 through 11, **characterized in that** a nasal adapter (26) for intranasal inhalation is either detachably or fixedly inserted into the mouthpiece (2).

13. The inhalation apparatus according to claim 12, **characterized in that** the nasal adapter comprises an olive protruding from the opening of the mouthpiece (2) that puts a flow duct against the nostril and seals it airtight.

14. The inhalation apparatus according to claims 12 and 13, **characterized in that** the nasal adapter (26) is held in the mouthpiece (2) using snap-in locking means that are configured to match specific nasal adapters with specific powder inhalers.

15. The inhalation apparatus according to any one of claims 1 through 11, **characterized in that** the cap (10) is longer than in the design without nasal adapter (26) and that the nasal adapter can be slid onto the mouthpiece (2).

16. The inhalation apparatus according to any one of claims 1 through 15, **characterized in that** the powder reservoir (12) is designed for a one-time filling of at least 200 doses.

17. The inhalation apparatus according to any one of claims 1 through 16, **characterized in that**, if the apparatus contains a hygroscopic powder drug, a desiccant that acts on it is provided in the apparatus.

18. The inhalation apparatus according to any one of claims 1 through 17, **characterized in that** the housing (1) accommodates a pump arrangement (21, 22) whose compressed air reservoir is connected to the dosing cavity (17) via a valve arrangement (7, 15a, 18, 25) or a diaphragm valve in such a way that the valve arrangement (7, 15a, 18, 25) releases the stored compressed air when the preloaded dosing ball (15) is released in sync with breathing from the movable stopper (14a).

19. The inhalation apparatus according to any one of claims 1 through 18, **characterized in that** the pump arrangement is formed by corrugated bellows (21) held on a movable base plate (22) and supported on the opposite side against a transverse wall (3) in the housing (1), and that the base plate (22) is joined with an angular lever (23) that serves as a means of load transmission and whose other end is connected to the tappet (24) of the operating lever (11), said corrugated bellows (21) being connected via an air outlet (4) with a compressed air duct (5) in the housing (1) and communicating via a radial axle end hole (25) with a hollow space in the axle end (6).

20. The inhalation apparatus according to any one of claims 1 through 19, **characterized in that** the valve arrangement for controlling the compressed air supply in sync with breathing from the corrugated bellows (21) into the air duct (27) is formed by a second radial axle end hole (25) and a first radial axle end bore (7), the wall of the bearing bore (15a) of the dosing ball (15) and a radial valve hole (18) in the dosing ball (15), said valve hole (18) being in true alignment with the first radial axle end bore (7) when the stop piece (20) is positioned at the bottom of the housing (1).

21. The inhalation apparatus according to any one of claims 1 through 20, **characterized in that** a bent blowpipe (19) whose opening points from outside into the direction of the dosing cavity (17) is connected to the valve hole (18) in the peripheral surface of the dosing ball (15).

22. The inhalation apparatus according to any one of claims 1 through 20, **characterized in that** all components except the torsion spring (16) are injection-molded plastic parts.

## Revendications

1. Appareil d'inhalation pour médicaments pulvérulents, constitué par un boîtier (1) comportant un dispositif de dosage (15) rotatif associé à l'ouverture d'un réservoir de poudre (12) se trouvant dans le boîtier, actionnable à la main et présentant une cavité de dosage périphérique pour recevoir une dose de poudre, comportant une surface périphérique en forme d'arc de cercle ainsi qu'un bec (2) raccordé au boîtier (1) au niveau du dispositif de dosage (15) et, à l'opposé de celui-ci, des orifices d'entrée d'air (4) pour former un canal d'air (5) dans lequel la dose de poudre libérée en tournant le dispositif de dosage (15) est dégagée dans le courant d'air inhalé d'un utilisateur, **caractérisé en ce que** le dispositif de dosage (15) est relié à des moyens de serrage (16) élastiques, présente un élément de butée (20) et peut être précontraint jusqu'à une butée (14a) mobile lorsque la cavité de dosage (17) reste dans la région de l'ouverture de prélèvement de poudre (12a) du réservoir à poudre (12), la butée (14a) pouvant être déplacée par le courant d'air inhalé par l'utilisateur pour dégager le dispositif de dosage (15) précontraint, et l'accélération ainsi déclenchée du dispositif de dosage (15) peut être subitement interrompue par l'élément de butée (20).

2. Appareil d'inhalation selon la revendication 1, **caractérisé en ce que** le dispositif de dosage est réalisé sous forme de bille de dosage (15) avec un alésage de montage (15a) centré et **en ce qu'**il est monté en rotation autour d'un tourillon (6).

3. Appareil d'inhalation selon les revendications 1 et 2, **caractérisé en ce que** le moyen de serrage élastique est un ressort à torsion (16) monté en rotation sur le tourillon avec une branche courte (16a) et une branche longue (16b), la branche courte (16a) étant reliée à la bille de dosage (15) et la branche longue (16b) étant reliée, via un trou oblong (9) en forme d'arc dans le boîtier (1), à un levier d'actionnement (11) monté en rotation sur la face extérieure du boîtier (1) et en alignement axial avec le tourillon (6), pour faire tourner et précontraindre la bille de dosage (15).

4. Appareil d'inhalation selon la revendication 3, **caractérisé en ce que** le levier d'actionnement (11) est guidé dans le trou oblong (9) via un goujon d'entraînement (24) et peut être arrêté dans la position précontrainte de la bille de dosage (15) par enclenchement ou par dépassement d'un point mort.

5. Appareil d'inhalation selon la revendication 1, **caractérisé en ce que** la butée (14a) mobile de manière synchrone à la respiration est montée sur un clapet de déclenchement (14) suspendu à pivotement dans le boîtier (1) sur le côté du bec, directement en amont de la bille de dosage (15), et fermant le canal de conduite d'air (27) et, lorsque la bille de dosage (15) est précontrainte, est en liaison active avec l'élément de butée (20) de celle-ci, la bille de dosage (15) étant agencée dans le canal de conduite d'air (27) de telle sorte que son élément de butée (20), après libération par la butée (14a) mobile sous l'effet de la force du ressort de torsion (16), est retenu sur le fond du boîtier (1) et que la cavité de dosage (17) est située en dehors de l'ouverture de prélèvement de poudre (12a).

6. Appareil d'inhalation selon les revendications 3 et 4, **caractérisé en ce que** sur l'extrémité libre du levier d'actionnement (11) est maintenu à déplacement un capuchon (10) qui, dans la position initiale du levier d'actionnement (11), peut être enfilé sur le bec (2) sur le boîtier (1).

7. Appareil d'inhalation selon la revendication 1, **caractérisé en ce que** le réservoir à poudre (12) est constitué par un matériau transparent et un regard (13) est prévu dans le boîtier (1) dans la région de la partie inférieure du réservoir à poudre (12).

8. Appareil d'inhalation selon les revendications 6 et 7, **caractérisé en ce que** sur le capuchon (10) est ménagée une plaque (10a) qui recouvre le regard (13) lorsque le capuchon (10) est enfilé.

9. Appareil d'inhalation selon l'une des revendications 1 à 8, **caractérisé en ce que** la cavité de dosage (17) est réalisée sous forme d'évidement en forme de segment sphérique.

10. Appareil d'inhalation selon l'une des revendications 1 à 9, **caractérisé en ce que** la bille de dosage est interchangeable et les billes de dosage peuvent être mises en oeuvre avec différentes tailles de cavité de dosage (17).

11. Appareil d'inhalation selon l'une des revendications 1 à 10, **caractérisé en ce qu'**un dispositif de secouage est raccordé au réservoir de poudre (12).

12. Appareil d'inhalation selon l'une des revendications 1 à 11, **caractérisé en ce que** pour l'inhalation intranasale, il est prévu un adaptateur nasal (26) inséré de manière amovible ou solidaire dans le bec (2).

13. Appareil d'inhalation selon la revendication 12, **caractérisé en ce que** l'adaptateur nasal présente une olive faisant saillie hors de l'ouverture du bec (2) avec un canal d'écoulement qui peut être appliqué de manière hermétique à la narine.

14. Appareil d'inhalation selon les revendications 12 et 13, **caractérisé en ce que** l'adaptateur nasal (26) est maintenu dans le bec (2) par des moyens d'enclenchement qui sont réalisés de telle sorte que des adaptateurs nasaux déterminés sont adaptés à des inhalateurs de poudre déterminés.

15. Appareil d'inhalation selon l'une des revendications 1 à 14, **caractérisé en ce que** le capuchon (10) est prolongé par rapport à la réalisation sans adaptateur nasal (26) et peut être enfilé via l'adaptateur nasal sur le bec (2).

16. Appareil d'inhalation selon l'une des revendications 1 à 15, **caractérisé en ce que** le réservoir à poudre (12) est réalisé pour un remplissage unique avec au moins 200 doses.

17. Appareil d'inhalation selon l'une des revendications 1 à 16, **caractérisé en ce que** de préférence dans le cas de médicament pulvérulent hygroscopique, il est prévu dans l'appareil un agent déshydratant agissant sur ce médicament.

18. Appareil d'inhalation selon l'une des revendications 1 à 17, **caractérisé en ce que** dans le boîtier (1) est logé un dispositif de pompe (21, 22) dont le réservoir d'air comprimé est branché à la cavité de dosage (17) via un dispositif à soupapes (7, 15a, 18, 25) ou via une soupape à membrane, de telle sorte qu'avec la libération synchrone à la respiration de la bille de dosage (15) depuis la butée (14a) mobile, le dispositif à soupapes (7, 15a, 18, 25) libère simultanément l'air comprimé accumulé.

19. Appareil d'inhalation selon l'une des revendications 1 à 18, **caractérisé en ce que** le dispositif de pompe est formé par un soufflet (21) maintenu sur une plaque de soutien (22) mobile et prenant appui de l'autre côté contre une paroi transversale (3) dans le boîtier (1), et **en ce que** la plaque de soutien (22) est reliée à un levier coudé (23) servant de moyen de transmission de force qui, à son extrémité opposée, est raccordé au boulon d'entraînement (24) du levier d'actionnement (11), le soufflet (21) étant en communication avec un espace creux réalisé dans le tourillon (6) via un orifice de sortie d'air (4) dans un canal d'air comprimé (5) dans le boîtier (1) et via un alésage de tourillon (25) radial.

20. Appareil d'inhalation selon l'une des revendications 1 à 19, **caractérisé en ce que** le dispositif de soupapes pour l'amenée d'air comprimé commandée de manière synchrone à la respiration hors du soufflet (21) jusque dans le canal de conduite d'air (27) est formé par le deuxième alésage de tourillon (25) radial et par un premier alésage de tourillon (7) radial, par la paroi de l'alésage de montage (15a) de la bille de dosage (15) et par un alésage de soupape (18) radial dans la bille de dosage (15), l'alésage de soupape (18) étant en alignement avec le premier alésage de tourillon (7) radial lorsque l'élément de butée (20) est en position de butée sur le fond du boîtier (1).

21. Appareil d'inhalation selon l'une des revendications 1 à 20, **caractérisé en ce qu'**à l'alésage de soupape (18) dans la surface périphérique de la bille de dosage (15) est raccordé un tube de soufflage (19) coudé dont l'ouverture est orientée depuis l'extérieur en direction de la cavité de dosage (17).

22. Appareil d'inhalation selon l'une des revendications 1 à 21, **caractérisé en ce que**, mis à part le ressort de torsion (16), tous les composants sont réalisés en matière plastique par moulage par injection.
